# EUROPÄISCHE PATENTANMELDUNG

(11) **EP 3 876 025 A1**
(43) Veröffentlichungstag der Anmeldung: **08.09.2021**
(21) Anmeldenummer: 20160433.7
(22) Anmeldetag: 02.03.2020
(51) Int. Cl.: G02C 11/00, G06F 1/16, A61B 5/11, G06Q 99/00

(54) **ANORDNUNG ZUR HINDERNISERKENNUNG UND KOLLISIONSWARNUNG**

(71) Anmelder: Siemens Aktiengesellschaft, 80333 München (DE)
(72) Erfinder: Kogard, Valentin, 2102 Hagenbrunn (AT); Pelzer, Alexander, 1100 Wien (AT); Winter, Richard, 1120 Wien (AT)
(74) Vertreter: Maier, Daniel Oliver

(57) **Zusammenfassung**

Die Erfindung betrifft eine Anordnung (AN) zum Erkennen von Hindernisobjekten und zum Warnen eines Anwenders vor einer Kollision mit einem Hindernisobjekt bzw. Objekt. Dabei kann die Anordnung (AN) an einem Körperteil, insbesondere dem Kopf, des Anwendern angebracht werden. Diese Anordnung (AN) umfasst dabei zumindest einen Umgebungssensor (US), insbesondere eine optische Sensoreinheit, zum Erfassen von Objekten in einer Umgebung des Anwenders sowie zwei Sensoreinheiten (S1, S2) zum Erfassen von Bewegungsparametern des Anwenders. Dabei weist die Anordnung (AN) eine Trägereinheit (TE) auf, an welcher zumindest der Umgebungssensor (US) und die zwei Sensoreinheiten (S1, S2) zum Erfassen von Bewegungsparametern angebracht sind. Weiterhin weist die Anordnung (AN) einen Auswerteeinheit (AW) auf, welche dazu eingerichtet ist:
- eine mittels des Umgebungssensors (US) erfasste Umgebungs- und/oder Objektinformation auszuwerten;
- auf Basis der mittels der zwei Sensoreinheiten (S1, S2) erfassten Bewegungsparameter eine Bewegungsinformation des Anwenders zu ermitteln;
- und die ausgewertete Umgebungs- und/oder Objektinformation mit der ermittelten Bewegungsinformation zu aggregieren und daraus Hinweise für den Anwender ableiten.

Außerdem umfasst die Anordnung (AN) zumindest eine Ausgabeeinheit (AE), durch welche dem Anwender Hinweise zu einem Hindernisobjekt und/oder Warnungen vor einer Kollision mit einem Objekt in seiner Umgebung ausgegeben werden können.

## Beschreibung

### Technisches Gebiet

Die vorliegende Erfindung betrifft eine Anordnung zum Erkennen von Hindernisobjekten und zum Warnen eines Anwenders vor einer Kollision mit einem Hindernisobjekt bzw. Objekt. Dabei kann die Anordnung an einem Körperteil, insbesondere dem Kopf, des Anwendern angebracht werden.

### Stand der Technik

Im industriellen Umfeld, insbesondere in einem Industrie-4.0-Umfeld, gewinnt Augmented Reality (kurz: AR) in Kombination mit einer Zusammenarbeit zwischen Mensch und Maschine bzw. Roboter immer mehr an Bedeutung. Unter Augmented Reality bzw. erweiterter Realität wird eine computerunterstützte Erweiterung der Realitätswahrnehmung, wobei darunter häufig nur eine visuelle Darstellung von Informationen - d.h. eine Ergänzung von Bildern und Videos mit computergenerierten Zusatzinformationen oder virtuellen Objekten mittels Einblendung und/oder Überlagerung verstanden wird. Im Industriebereich kann AR beispielsweise in der Fertigung und Produktion (z.B. Abgleichen digitaler Planungsdaten mit vorhandenen, realen Geometrien, Absicherungsmethoden durch Kombination von digitalen Planungsdaten mit realen Prototypen bzw. Konstruktionen, etc.), bei der Montage (z.B. Einblenden eines nächsten Arbeitsschritts direkt ins Sichtfeld des jeweiligen Anwenders, etc.) oder in der Wartung (z.B. Einblenden von Plänen, etc.) eingesetzt werden. Dabei spielt die Mensch-Maschine-Kollaboration in der Zusammenarbeit mit Transportdrohen, selbstfahrenden, fahrerlosen Transportfahrzeugen - so genannten Automatic Guided Vehicles oder AGV - und Robotern in verschiedenen Anwendungen eine immer wichtiger werdende Rolle.

Insbesondere wenn so genannte Virtual Reality (VR)-Vorrichtungen (z.B. VR-Brille, VR-Headset, etc.) für AV-Anwendungen zum Einsatz kommen, kann die Umgebung zumindest teilweise, wenn nicht komplett ausgeblendet sein. Da die virtuelle Realität zumindest nur teilweise in Zusammenhang mit der tatsächlich den Anwender umgebenen Realität und den Objekten stehen muss, kann ein Anwender von Augemented Realität bzw. ein Nutzer einer Virtual Reality-Vorrichtung, wie z.B. einer VR-Brille oder VR-Headset, nur bedingt erkennen, dass Objekte bzw. Hindernisse sich in seiner realen Umgebung befinden bzw. sich in dieser bewegen, wie z.B. AGVs, Roboterteil/-arme, etc. Daher ist es notwendig, Sicherheitskonzept zu entwickeln, durch welche Menschen, welche unter Nutzung von VR- und AV-Konzepten mit Maschinen (z.B. Robotern, Transportdrohen, AGVs) zusammenarbeiten, vor möglichen Gefahren und Verletzungen durch Kollisionen mit Hindernisobjekte zu schützen.

Aus den Schriften EP 2 490 155 A1, EP 2 629 242 A1, US 2018/0218643 A1 oder WO 2014/140843 A1 sind beispielsweise tragbare Vorrichtungen vorwiegend zur Unterstützung von sehbehinderten Menschen bekannt, welche über einen Sensor Bilder der Umgebung im Gesichtsfeld eines Anwenders aufnehmen. Diese Bilder werden mittels einer Verarbeitungseinheit klassifiziert bzw. mit in einer Datenbank abgespeicherten Bilder von Objekten, Personen, Gesten, Texten, etc. verglichen, um dem Anwender Informationen über erkannte Objekte, Personen, Gesten, Texten, etc. zur Verfügung zu stellen. Die beschriebene Vorrichtung weist allerdings den Nachteil auf, dass die Vorrichtung trainiert werden muss, um Objekten, Personen, Gesten, Texten, etc. zu erkennen und damit nur Informationen über bereits bekannte bzw. in der Datenbank hinterlegte Objekte, Personen, etc. ausgeben kann. Allerdings kann mit derartigen Vorrichtungen keine Annäherung zwischen einem möglichen Hindernisobjekt und dem Anwender - insbesondere in einer beliebigen Umgebung - festgestellt und damit keine sichere Kollisionswarnung des Anwenders vorgenommen werden. Weiterhin muss die Funktion der Vorrichtung vom Anwender mittels Knopfdrucks aktiviert werden, wodurch einen Echtzeiterkennung von etwaigen Hindernissen nicht gewährleistet ist.

Im Bereich der Industrie 4.0 gibt es beispielsweise - wie z.B. aus den Schriften EP 2 508 956 B1 oder EP 3 364 263 A1 bekannt - Verfahren zur Kollisionsvermeidung zwischen selbstfahrenden, fahrerlosen Transportfahrzeugen (AGVs) bzw. zur Überwachung eines Fahrweges derartiger Vehikel. Dabei sind allerdings die geplanten Fahrwege der AGVs innerhalb eines vorbestimmten Gebietes (z.B. Fabrikhalle, etc.) vorgegeben und werden auf Überschneidungen und Kollisionsgefahren untersucht, um bei einer Kollision z.B. durch Veränderung des Fahrwegs, Anhalten des AGVs, etc. zu vermeiden. Als Basis der Hinderniserkennung werden z.B. Laser, Barcodes, Magnetbänder, induktive Leiter oder die in einem übergeordneten System bekannte Navigation der AGVs verwendet. Derartige Verfahren und Systeme sind daher nicht in einer beliebigen Umgebung einsetzbar und bedürfen einer relativ genauen Fahrwegplanung.

Weiterhin ist aus der Veröffentlichung "RUBIN Wissenschaftsmagazine; Seite 28 bis 31; Nr. 2/2019" der Ruhr-Universität Bochum eine Augmented Reality-Anwendung bekannt, welche im Bauwesen bzw. in der Gebäudetechnik als digitaler Wartungshelfer eingesetzt werden kann. Die dafür entwickelten Algorithmen sollen beispielsweise ermöglichen, dass der digitale Wartungshelfer, welcher z.B. in Form von so genannten "Smart Glasses" ausgeführt ist, künftig in Echtzeit und automatisch (d.h. ohne aktive Kalibrierung) erkennt, an welcher Position oder gesamten Gebäude er sich in einem Raum befindet. Dabei wird vom digitalen Wartungshelfer bzw. einem zugehörigen Algorithmus ein von einer Kamera aufgezeichnetes Bild mit einem digitalen Modell des Gebäudes verglichen. Damit weist der digitale Wartungshelfer allerdings ebenfalls den Nachteil auf, nicht in einer beliebigen Umgebung einsetzbar zu sein. Er kann beispielsweise nur in einer Umgebung bzw. in einem Gebäude für z.B. Planung oder Wartung genutzt werden, von welchem vorab ein dreidimensionales Modell erstellt wurde.

### Darstellung der Erfindung

Der Erfindung liegt daher die Aufgabe zugrunde, eine Anordnung anzugeben, durch welche in einer beliebigen Umgebung rasch und auf einfache Weise Hindernisse erkannt, ein Anwender der Anordnung vor Kollisionen mit diesen Hindernissen gewarnt und dadurch Unfalls- und Verletzungsrisiko für den Anwender reduziert wird.

Diese Aufgabe wird durch eine Anordnung eingangs angeführter Art mit den Merkmalen gemäß dem unabhängigen Patentanspruch gelöst. Vorteilhafte Ausführungsformen der vorliegenden Erfindung sind in den abhängigen Ansprüchen beschrieben.

Erfindungsgemäß erfolgt die Lösung der Aufgabe durch eine Anordnung, welche an einem Körperteil, insbesondere einem Kopf eines Anwenders, angebracht werden kann. Diese Anordnung umfasst zumindest:
- einen Umgebungssensor zum Erfassen von Objekten in einer Umgebung des Anwenders;
- zwei Sensoreinheiten, welche unabhängig voneinander Bewegungsparametern des Anwenders erfassen;
- eine Trägereinheit, an welcher zumindest der Umgebungssensor und die zwei Sensoreinheiten zum Erfassen von Bewegungsparametern angebracht sind;
- eine Auswerteeinheit, welche dazu eingerichtet ist:
   o eine mittels des Umgebungssensors erfasste Umgebungs- und/oder Objektinformation auszuwerten;
   o auf Basis der mittels der zwei Sensoreinheiten erfassten Bewegungsparameter eine Bewegungsinformation des Anwenders zu ermitteln;
   o und die ausgewertete Umgebungs- und/oder Objektinformation mit der ermittelten Bewegungsinformation zu aggregieren und daraus Hinweise für den Anwender ableiten; und
- zumindest eine Ausgabeeinheit, welche derart eingerichtet ist, dass dem Anwender Hinweise ausgegeben werden können.

Der Hauptaspekt der gegenständlichen Erfindung besteht darin, dass die erfindungsgemäße Anordnung rasch und ohne zusätzlichen Aufwand in einer beliebigen Umgebung einsetzbar ist. Dies wird insbesondere durch eine kombinierte Auswertung einer Information über die Umgebung, welche mittels eines Umgebungssensor erfasst wird, und einer Bewegungsinformation des Anwenders, welche auf Basis von mit Hilfe von zwei Sensoreinheiten erfassten Bewegungsparametern ermittelt wird, erreicht. Die Anordnung muss damit nicht auf eine neue Umgebung angelernt bzw. trainiert werden, um Hindernisse zu erkennen und/oder den Anwender von möglichen Kollisionen zu warnen. Die erfindungsgemäße Anordnung kann damit sehr einfach von sehbehinderten oder blinden Menschen verwendet werden, um sich z.B. in einer Umgebung zu orientieren oder Hindernisse in alltäglichen Leben zu erkennen bzw. Kollisionen mit Objekten zu vermeiden. Weiterhin kann die Anordnung sehr einfach in Kombination mit Virtual Reality- bzw. Augmented Reality-Anwendung im Bereich der Industrie 4.0 eingesetzt werden, um zu helfen Hindernisse z.B. im Bereich Entwicklung, Produktion und Fertigung zu erkennen und einen Benutzer der Anordnung vor diesen zu warnen. Dadurch kann ein Verletzungs- und Unfallsrisiko auf einfache Weise reduziert werden.

Es ist weiterhin vorteilhaft, wenn die Auswerteeinheit der Anordnung dazu eingerichtet ist, ein Auswerten der erfassten Umgebungs- und/oder Objektinformation, eine Ermittlung der Bewegungsinformation des Anwenders aus den erfassten Bewegungsparametern, eine Aggregation der ausgewerteten Umgebungs- und/oder Objektinformation mit der ermittelten Bewegungsinformation und ein Ableiten der Hinweise für den Anwender in Echtzeit durchführen kann. Damit kann ein Anwender der Anordnung rasch vor z.B. herannahenden Objekten (z.B. Maschinenteilen, wie z.B. Roboterarmen, AGV, etc.) gewarnt und eine Kollision rechtzeitig verhindert werden. Weiterhin erhält der Anwender immer zeitaktuell Informationen über Objekte bzw. Hindernisse in seiner aktuellen Umgebung.

Es ist weiterhin günstig, wenn die zwei Sensoreinheiten zum Erfassen von Bewegungsparametern jeweils dazu eingerichtet sind, als Bewegungsparameter zumindest jeweils Lagewerte, Beschleunigungswerte und Drehbewegungswerte für die Ermittlung der Bewegungsinformation des Anwenders zu erfassen. Auf Basis dieser Werte kann eine Bewegungsinformation des Anwenders auf einfache Weise ohne manuellen Kalibrierungsaufwand und mit einer geringen Fehlerrate ermittelt werden. Weiterhin wird dadurch verhindert, dass beispielsweise pausenlos auf entsprechende Hindernisse hingewiesen wird, da auf Basis dieser als Bewegungsparameter erfassten Werte eine Bewegungsinformation des Anwenders ermittelt werden kann, anhand welcher beispielsweise festgestellt werden kann, ob und in welche Richtung sich der Anwender bewegt.

Bei einer bevorzugten Ausgestaltung der Erfindung ist die Auswerteeinheit zum Ermitteln der Bewegungsinformation des Anwenders auf Basis der mittels der zwei Sensoreinheiten erfassten Bewegungsparameter derart eingerichtet, dass auf Basis der Beschleunigungswerte, welche von jeder der zwei Sensoreinheiten unabhängig voneinander erfasst werden, die Bewegungsinformation des Anwenders ermittelt wird. Diese Bewegungsinformation kann beispielsweise in Form eines Bewegungsoder Beschleunigungsvektors z.B. mit Betrag und Bewegungswinkel in einem dreidimensionalen Raum angegeben werden, wobei der Betrag des Vektors z.B. angibt, ob sich der Anwender bewegt, und der Bewegungswinkel z.B. eine Information liefert, in welche Richtung die Bewegung des Anwenders erfolgt. Dabei wird mit Hilfe der von den zwei Sensoreinheiten unabhängig voneinander erfassten Lagewerten eine Wirkung der Erdbeschleunigung aus den erfassten Beschleunigungswerten herausgerechnet - d.h. die Erdbeschleunigung bzw. Gravitation wird von den Beschleunigungswerten, die von der jeweiligen Sensoreinheit erfasst wird, abgezogen, um in einem "schwerlosen Raum" rechnen zu können und die Bewegungen des Anwenders unabhängig von der wirkenden Erdanziehung darstellen zu können. Weiterhin wird die ermittelte Bewegungsinformation, insbesondere der auf Basis der Beschleunigungswerte der zwei Sensoreinheiten ermittelte Bewegungswinkel, nur dann zum Aggregieren mit der ausgewertete Umgebungs- und/oder Objektinformation genutzt, wenn von einem Wert, für dessen Bildung jeweils zumindest ein von jeder der zwei Sensoreinheiten erfasster Drehbewegungswert verwendet wird, ein vordefinierter Grenzwert überschritten wird. Zum Erfassen von Drehbewegungswerten kann jeder der zwei Sensoreinheiten einen Gyrosensor aufweisen, durch welchen Lageveränderungen und Drehbewegungen, insbesondere eine Drehbewegung des Kopfes des Anwenders festgestellt werden können. Nur wenn ein Wert, welcher auf Basis der von den beiden Sensoreinheiten erfassten Drehbewegungswerten ermittelt wurde, einen vordefinierten Grenzwert übersteigt, wird die ermittelte Bewegungsinformation, vor allem der ermittelte Bewegungswinkel, für die Aggregation genutzt. Liegt dieser Wert unterhalb des vordefinierten Grenzwerts, so wird die Bewegungsinformation bzw. der Bewegungswinkel auf einen Wert Null gesetzt, um z.B. Fehlmessungen, Störungen bei der Erfassung der Bewegungsparameter, fälschliche Winkelausgaben, etc. zu vermeiden.

Idealerweise ist die Auswerteeinheit der Anordnung auch dazu eingerichtet ist, aus den mittels der zwei Sensoreinheiten erfassten Bewegungsparametern wiederholt in einem vorgegebenen zeitlichen Abstand eine Bewegungsinformation des Anwenders zu ermitteln. Die Bewegungsinformation kann beispielsweise immer in einem zeitlichen Abstand von einigen Millisekunden (z.B. 3 ms) bestimmt werden und damit die Bewegungsinformation laufend an eine mögliche Bewegung des Anwenders angepasst.

Zweckmäßigerweise sind die zwei Sensoreinheiten zum Erfassen von Bewegungsparametern derart auf der Trägereinheit angebracht, dass sie symmetrisch zu einer Mittelachse der Trägereinheit positioniert sind. Insbesondere bei einer Trägereinheit, welche am Kopf des Anwenders tragbar ist (z.B. Brille, Stirnband, helmförmige Vorrichtung), können die zwei Sensoreinheiten zum Erfassen von Bewegungsparametern beispielsweise symmetrisch zu einer Drehachse des Kopfes um einen zum Körper des Anwenders senkrecht orientiert Achse auf der Trägereinheit positioniert sein. D.h. die Sensoreinheiten sind beim Tragen der Anordnung bzw. der Trägereinheit in Nähe bzw. bei den Ohren des Anwenders platziert und liegen damit annähernd symmetrisch bzw. im gleichen Abstand zur Drehachse des Kopfes, welche durch den ersten Halswirbel "Atlas" verläuft. Durch eine derartige Positionierung der Sensoreinheiten kann die Bewegungsinformation des Anwenders, welcher zuerst für jede Sensoreinheit getrennt bestimmt und dann kombiniert wird, mit einfachen Rechenoperationen ermittelt werden. Durch einen geringen Rechenaufwand kann die Anordnung damit energieschonend arbeiten.

Bei einer bevorzugten Ausgestaltung der erfindungsgemäßen Anordnung ist die Auswerteeinheit auf der Trägereinheit mittels einer lösbaren Verbindung angebracht. Damit kann die Anordnung auf einfache Weise an einem Körperteil - z.B. am Kopf - vom Anwender getragen werden. Alternativ kann die Auswerteeinheit auch als eigenständige Einheit, welche an einer anderen Position am Körper vom Anwender getragen werden kann, ausgeführt sein. Dadurch kann z.B. ein Gewicht und/oder eine Größe der Trägereinheit reduziert werden. Bei beiden Varianten ist die Auswerteeinheit mit dem Umgebungssensor und den beiden Sensoreinheiten zum Erfassen von Bewegungsparametern idealerweise über eine drahtgebundene Verbindung lösbar verbunden. Die drahtgebundene Verbindung dient für eine rasche Übertragung mit möglichst geringen zeitlichen Verlusten der vom Umgebungssensor bzw. den beiden Sensoreinheiten zum Erfassen von Bewegungsparametern erfassten Daten und Informationen, sodass die Auswertung möglichst ohne zeitliche Verlust durchgeführt werden kann.

Eine zweckmäßige Ausgestaltung der Erfindung sieht vor, dass die Ausgabeeinheit als akustische Ausgabeeinheit ausgestaltet ist, über welche die Hinweise für den Anwender in Form von vordefinierten Audiotexten und/oder Warnsignalen ausgegebenen werden können. Die Ausgabeeinheit kann dazu beispielsweise einen Lautsprecherteil umfassen, über welche die vordefinierten Audiotexte und/oder Warnsignale bei Erkennen von Hindernissen oder einer Kollisionsgefahr ausgegeben werden. Weiterhin ist es günstig, wenn die Ausgabeeinheit auf der Trägereinheit angebracht ist. Die Ausgabeeinheit kann dabei z.B. lösbar oder fest mit der Trägereinheit verbunden sein bzw. in die Trägereinheit integriert (z.B. integrierter Lautsprecher) sein. Zusätzlich kann die Ausgabeeinheit über eine drahtgebundene oder drahtlose Kommunikationsverbindung mit der Auswerteeinheit verbunden sein. Über die Kommunikationsverbindung können beispielsweise die vordefinierten Audiotexte und/oder Steuersignale und Anweisungen zum Ausgeben der vordefinierten Audiotexte bzw. von Warnsignalen übertragen werden.

Es ist weiterhin günstig, wenn der Umgebungssensor der Anordnung einen optischen Sensor, insbesondere Kamera und/oder Wärmekamera, umfasst. Mittels des optischen Sensors kann auf einfache Weise laufend einen Umgebungs- und/oder Objektinformation in Form von Bildinformationen aufgenommen werden, welche z.B. von der Auswerteeinheit mit Hilfe einer Objekterkennungssoftware auf einfache Weise ausgewertet wird. Alternativ kann der Umgebungssensor auch einen Radar- oder Ultraschallsensor zum Erfassen einer Umgebungs-/Objektinformation aufweisen. Derartige Sensoren weisen allerdings gegenüber einem optischen Sensor, insbesondere einer Kamera, den Nachteil auf, dass farbliche und/oder physikalische Eigenschaften von erfassten Objekten nicht differenziert und auch z.B. einfache Linien nicht detektiert werden können.

Es ist besonders von Vorteil, wenn die Trägereinheit beispielsweise brillenförmig, helmförmig oder als Stirnband ausgestaltet ist. Derartige Ausgestaltungen können von einem Anwender auf einfache Weise am Kopf angebracht und getragen werden. Weiterhin sind derartige Trägereinheit z.B. einfach auf Kunststoff herstellbar bzw. bereits verfügbar. Vor allem bei einer brillenförmigen Trägereinheit kann z.B. eine bereits für andere Anwendungen genutzte Brille (z.B. VR-Brille) für die erfindungsmäßige Anordnung mitbenutzt werden.

### Kurzbeschreibung der Zeichnung

Die Erfindung wird nachfolgend in beispielhafter Weise anhand der beigefügten Figuren erläutert. Dabei zeigen:
- Figur 1: beispielhaft und schematisch einen Aufbau der erfindungsgemäßen Anordnung zum Erkennen von Hindernisobjekten und zum Warnen eines Anwenders vor Kollisionen
- Figur 2: schematisch und beispielhaft eine vorteilhafte Ausführungsform der erfindungsgemäßen Anordnung zum Erkennen von Hindernisobjekten und zum Warnen eines Anwenders vor Kollisionen

### Ausführung der Erfindung

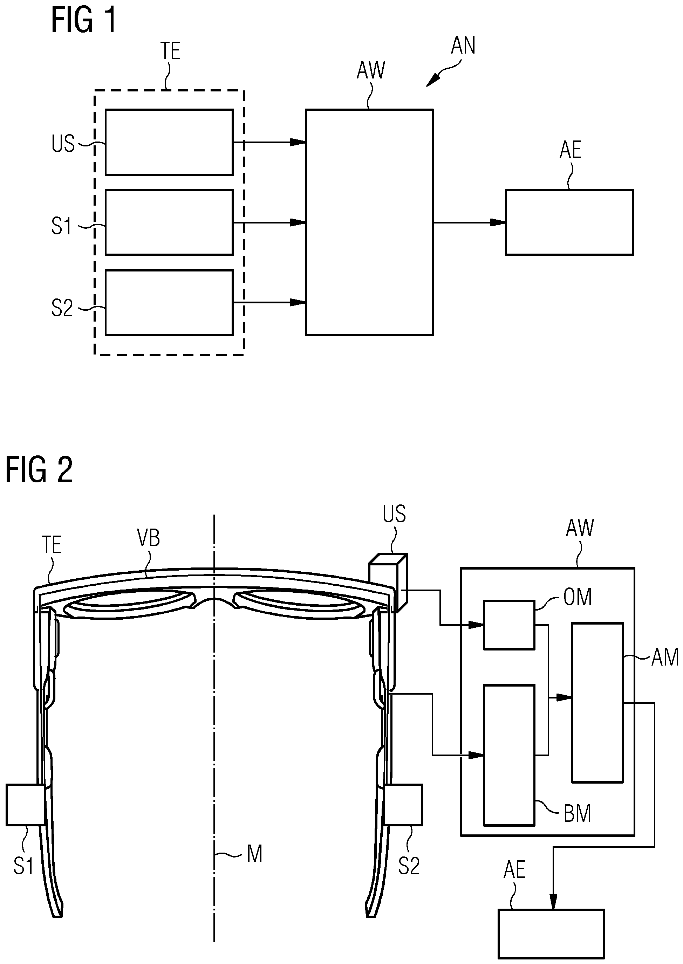

Figur 1 zeigt beispielhaft in schematischer Weise einen Aufbau der erfindungsgemäßen Anordnung AN zum Erkennen von Hindernisobjekten in einer Umgebung eines Anwenders bzw. zum Warnen eines Anwenders vor möglichen Kollisionen mit zumindest einem Objekt. Die Anordnung kann dazu mittels einer Trägereinheit TE an einem Körperteil, insbesondere einem Kopf des Anwenders angebracht werden. Dazu kann die Trägereinheit TE beispielsweise brillenförmig, helmförmig oder in Form eines Stirnbandes ausgestaltet sein. Es sind allerdings auch andere Ausführungsformen der Trägereinheit TE denkbar, welche in vorteilhafter Weise ein einfaches Anbringen der erfindungsgemäßen Anordnung AN an einem Körperteil des Anwenders, vor allem dem Kopf, ermöglichen.

Weiterhin umfasst die Anordnung AN zumindest einen Umgebungssensor US sowie zwei Sensoreinheiten S1, S2 zum Erfassen von Bewegungsparametern des Anwenders. Der Umgebungssensor US und die zwei Sensoreinheiten S1, S2 zum Erfassen von Bewegungsparametern sind dazu an der Trägereinheit TE angebracht.

Mit dem Umgebungssensor US können Objekte in der Umgebung des Anwenders erfasst werden. Dazu umfasst der Umgebungssensor US zumindest einen optischen Sensor, wie z.B. eine Kamera und/oder Wärmekamera. D.h. der Umgebungssensor US erfasst mit dem optischen Sensor z.B. laufend eine optische Information der Umgebung und/oder der Objekte in der Umgebung des Anwenders. Bei einer vorteilhaften Ausführungsform der Anordnung AN, bei welcher die Trägereinheit TE am Kopf des Anwenders anbringbar ist, kann der Umgebungssensor US derart an der Trägereinheit TE angeordnet sein, dass der Umgebungssensor US beispielsweise auf der Stirn bzw. über zumindest einem Auge oder in der Nähe eines Auges des Anwenders positioniert ist.

Die beiden Sensoreinheiten S1, S2 zum Erfassen von Bewegungsparametern des Anwenders sind ebenfalls an der Trägereinheit TE angebracht. Bei einen am Kopf getragenen Trägereinheit TE (z.B. Brille, Stirnband, helmförmige Vorrichtung) können die Sensoreinheiten S1, S2 symmetrisch zu einer Mittelachse der Trägereinheit TE angeordnet sein, wobei die Positionierung derart gewählt wird, dass beim Tragen der Anordnung AN durch den Anwender die Sensoreinheiten S1, S2 möglichst nahe bei den Ohren positioniert sind. D.h. eine erster Sensoreinheit S1 kommt z.B. beim linken Ohr und eine zweite Sensoreinheit S2 z.B. beim rechten Ohr des Anwenders zum Liegen. Da die Sensoreinheiten S1, S2 sich bei einer derartigen Positionierung annähernd auf einer Drehachse des Kopfes über dem ersten Halswirbel Altas befinden, kann eine Bewegung des Anwenders bzw. eine Bewegungsinformation des Anwenders ohne großen Rechenaufwand detektiert bzw. ermittelt werden. Weiterhin sind die erste Sensoreinheit S1 wie die zweite Sensoreinheit S2 dazu eingerichtet, für eine Detektion der Bewegung des Anwenders laufend Bewegungsparameter - wie beispielsweise Lagewerte, Beschleunigungswerte und Drehbewegungswerte - unabhängig voneinander zu erfassen. D.h. die erste wie die zweite Sensoreinheit S1, S2 erfasst jeweils die von ihr festgestellten Bewegungsparameter. Als erste und zweite Sensoreinheiten S1, S2 können beispielsweise am Markt verfügbare Bewegungs- oder Beschleunigungssensoreinheiten verwendet werden, welche beispielsweise einen Beschleunigungssensor zum Erfassen von Beschleunigungswerten, einen Lagesensor zum Erfassen von Lagewerten und einen Gyrosensor zum Erfassen von Lageveränderungen und Drehbewegungen als Drehbewegungswerte aufweisen.

Weiterhin umfasst die erfindungsgemäße Anordnung AN eine Auswerteeinheit AW, welche für eine Übertragung der mit dem Umgebungssensor US und den zwei Sensoreinheiten S1, S2 erfassten Information, Daten und Parametern eine drahtgebundene Verbindung aufweist, welche als lösbare Verbindung (z.B. Steckverbindung, Kabelverbindung zum Einstecken, etc.) ausgestaltet sein kann. Die Auswerteeinheit AW kann beispielsweise lösbar auf der Trägereinheit TE angebracht sein kann. D.h. die Auswerteeinheit AW kann z.B. mittels einer Steckverbindung, welche auch die Verbindung zum Umgebungssensor US und den zwei Sensoreinheiten S1, S2 herstellt, auf der Trägereinheit lösbar angebracht werden. Alternativ kann die Auswerteeinheit AW z.B. als eigenständige Einheit ausgestaltet sein, welche beispielsweise über eine Kabelsteckverbindung mit der Trägereinheit TE bzw. den darauf angebrachten Sensoreinheiten S1, S2 und dem Umgebungssensor US verbunden wird, und vom Anwender z.B. an einer beliebigen Stelle am Körper (z.B. Gürtel, Jackentasche, etc.) getragen werden kann.

Die Auswerteeinheit AW ist außerdem dazu eingerichtet, die Objekt- und/oder Umgebungsinformation, welche vom Umgebungssensor US erfasst wird, auszuwerten. Dazu kann von der Auswerteeinheit AW beispielsweise eine Objekterkennungssoftware eingesetzt werden. Bei der Auswertung kann z.B. eine Annäherung zwischen einem Objekt bzw. Hindernisobjekt und dem Anwender erkannt werden.

Weiterhin ist die Auswerteeinheit AW dazu eingerichtet, auf Basis der laufende erfassten Bewegungsparameter - z.B. Lagewerte, Beschleunigungswerte und Drehbewegungswerte - eine Bewegungsinformation des Anwenders zu ermitteln. Dazu wird zuerst auf Basis der Beschleunigungswerte, welche von jeder der zwei Sensoreinheiten S1, S2 unabhängig voneinander erfasst werden, die Bewegungsinformation ermittelt. Dabei kann beispielsweise zuerst eine Bewegungsinformation für die erste Sensoreinheit S1 und eine Bewegungsinformation für die zweite Sensoreinheit S2 getrennt voneinander bestimmt werden. Diese Bewegungsinformationen können beispielsweise in Form eines Bewegungs- oder Beschleunigungsvektors z.B. durch Vektorlänge bzw. Betrag und Angabe eines Bewegungswinkels in einem 3-dimensionalen Koordinatensystem angegeben werden. Für die Bestimmung der Bewegungsinformationen der ersten und der zweiten Sensoreinheit S1, S2 wurden mit Hilfe der von der jeweiligen Sensoreinheit S1, S2 erfassten Lagewerte eine Erdbeschleunigung bzw. Gravitation aus den jeweils erfassten Beschleunigungswerten herausgerechnet, um eine Bewegung des Anwenders unabhängig von der wirkenden Erdanziehung darzustellen. Die für die zwei Sensoreinheiten S1, S2 getrennt ermittelte Bewegungsinformationen werden dann miteinander kombiniert, um eine Gesamtbewegungsinformation der Anordnung AN für die Weiterverarbeitung durch die Auswerteeinheit zu AW erhalten.

Im einfachsten Fall können beispielsweise die Bewegungsinformation der ersten Sensoreinheit S1 und die Bewegungsinformation der zweiten Sensoreinheit S2 addiert und gemittelt werden. D.h. es werden beispielsweise die Beträge der für die zwei Sensoreinheiten ermittelten Bewegungs- oder Beschleunigungsvektoren addiert und gemittelt und die zugehörigen Winkelangaben entsprechend zusammengeführt und z.B. von Radiant in Grad umgerechnet. In der Bewegungsinformation ist dann beispielsweise durch die Vektorlänge bzw. den Betrag die Information enthalten, ob der Anwender bzw. die Anordnung AN sich bewegt. Die Angabe des Bewegungswinkels im dreidimensionalen Raum bzw. Koordinatensystem gibt beispielsweise an, in welche Richtung diese Bewegung durchgeführt wird. Idealerweise werden zur Ermittlung der Bewegungsinformation Mittelwerte über eine vorgebbare Anzahl an von der jeweiligen Sensoreinheit S1, S2 erfassten Beschleunigungs- und Lagewerten gebildet (z.B. ein Mittelwert über die letzten 25 erfassten Werte) und die Bewegungsinformation auf Basis dieser Mittelwerte bestimmt, damit einzelne Fehlmessungen (z.B. Peaks) der Sensoreinheiten S1, S2 nicht zu gravierenden Fehlern in der Auswertung führen.

Die Ermittlung der Bewegungsinformation kann beispielsweise laufend in einem zeitlich vorgegebenen Abstand (z.B. alle 3 Millisekunden) wiederholt werden. Auf diese Weise kann rasch auf eine Veränderung in der Umgebung und/oder Bewegungsveränderung des Anwenders reagiert werden.

Um eine fälschliche Winkelausgabe bzw. eine fälschliche Winkelangabe in der Bewegungsinformation zu vermeiden, werden von der Auswerteeinheit AW die von den zwei Sensoreinheiten S1, S2 erfassten Drehbewegungswerte verwendet. Dazu kann beispielsweise aus zumindest jeweils einem Drehbewegungswert der erste Sensoreinheit S1 und zumindest jeweils einem Drehbewegungswert der zweiten Sensoreinheit S2 ein Wert (z.B. Mittelwert) gebildet werden. Dieser Wert wird dann mit einem vordefinierten Grenzwert verglichen. Wird dieser Grenzwert vom aus den Drehbewegungswerten gebildeten Wert überschritten, so wird die Bewegungsinformation, insbesondere die ermittelte Winkelangabe für eine Weiterverarbeitung durch die Auswerteeinheit AW genutzt. Wird dieser Grenzwert vom aus den Drehbewegungswerten gebildeten Wert nicht überschritten, so wird die Bewegungsinformation bzw. die ermittelte Winkelangabe z.B. auf den Wert Null gesetzt. Der vordefinierte Grenzwert kann einerseits statisch - beispielsweise auf Basis von Erfahrungswerten - festgelegt werden oder andererseits dynamisch ermittelt werden. Bei einer dynamischen Ermittlung kann z.B. ein Mittelwert über eine vorgegebenen Anzahl an Drehbewegungswerten (z.B. über die letzten 100 Werte) der beiden Sensoreinheiten S1, S2 gebildet werden. Dadurch wird der Grenzwert beispielsweise langsam an einen neuen Grenzwert angepasst.

Die ausgewertete Umgebungs- und/oder Objektinformation wird dann mit der ermittelten Bewegungsinformation des Anwenders von der Auswerteeinheit AW verknüpft bzw. aggregiert werden. Dabei kann beispielsweise die Bewegungsinformation nur dann verarbeitet werden, wenn z.B. anhand der ausgewerteten Umgebungs- und/oder Objektinformation festgestellt wird, dass eine Annäherung zwischen einem Objekt und dem Anwender vorliegt. Eine Auswertung der mit dem Umgebungssensor US erfassten Umgebungs- und/oder Objektinformation und eine Ermittlung der Bewegungsinformation des Anwender auf Basis der von den Sensoreinheiten S1, S2 erfassten Bewegungsparameter erfolgt parallel und in Echtzeit. Die Aggregation und Verarbeitung von ausgewertete Umgebungs- und/oder Objektinformation und ermittelter Bewegungsinformation des Anwenders durch die Auswerteeinheit AW erfolgt ebenfalls in Echtzeit. Aus der Aggregation der ausgewertete Umgebungs- und/oder Objektinformation und der ermittelten Bewegungsinformation des Anwenders kann von der Auswerteeinheit AW ein Hinweis für den Anwender ausgegeben werden, dass z.B. ein Hindernisobjekt vorhanden ist, eine Kollisionsgefahr mit einem Objekt besteht, etc.

Für die Ausgabe von Hinweisen umfasst die Anordnung AN weiterhin zumindest eine Ausgabeeinheit AE. Die Ausgabeeinheit AE kann beispielsweise als akustische Ausgabeeinheit AE ausgestaltet sein, über welche Hinweise zu in der Umgebung befindlichen Hindernissen und/oder Objekten in Form von vorgefertigten Audiotexten ausgegeben werden können, durch welche z.B. dem Anwender verbal Hindernisobjekt- bzw. Gefahrenpositionen beschrieben werden können. Alternativ oder zusätzlich, können z.B. bei Kollisionsgefahr des Anwenders mit einem Objekt Warnsignale über die Ausgabeeinheit AE ausgegebenen werden. Die Ausgabeeinheit AE kann beispielsweise ebenfalls auf der Trägereinheit TE festverbunden - z.B. in Form von integrierten Kopfhörern - oder lösbar - z.B. als lösbar anbringbare Kopfhörer - angebracht sein. Alternativ kann die Ausgabeeinheit als eigenständige Einheit (z.B. tragbare Lautsprechereinheit, etc.) ausgeführt sein, welche über eine drahtgebundene oder drahtlose Kommunikationsverbindung (z.B. Kabel, Funk, etc.) mit der Auswerteeinheit AW verbindbar ist. Über die Kommunikationsverbindung können beispielsweise Steuersignalen zur Ausgabe der Hinweise und/oder zum Übertragen von Daten (z.B. vordefinierte Audiotexte, etc.) von der Auswerteeinheit AW zur Ausgabeeinheit AE übertragen werden. Es ist weiterhin denkbar, dass die Ausgabeeinheit AE mit der Auswerteeinheit AW festverbunden oder in diese integriert ist - z.B. in Form einer in die Auswerteeinheit integrierten Lautsprechereinheit.

In Figur 2 ist beispielhaft und schematisch eine besonders vorteilhafte Ausführungsform der erfindungsgemäßen Anordnung AN zum Erkennen von Hindernisobjekten und zum Warnen eines Anwenders vor Kollisionen dargestellt. Die Trägereinheit TE weist dabei eine Brillenform auf, auf welcher zumindest der Umgebungssensor US und die zwei Sensoreinheiten S1, S2 zum Erfassen von Bewegungsparametern angebracht sind. Der Umgebungssensor US ist dabei z.B. an einem linken oder rechten Außenrand der Brillenform bzw. des Augenteils der Brillenform derart angebracht, dass der Umgebungssensor beim Tragen der brillenförmigen Trägereinheit TE in der Nähe eines der Augen des Anwenders positioniert ist. Alternativ könnte der Umgebungssensor US aus zwischen den Augen des Anwenders z.B. in der Mittelachse M über dem Nasenteil der brillenförmigen Trägereinheit TE positioniert sein.

Die beiden Sensoreinheiten S1, S2 zum Erfassen der Bewegungsparameter sind z.B. symmetrisch zur Mittelachse M der brillenförmigen Trägereinheit TE z.B. seitlich an den Bügeln der Brillenform positioniert. Dabei ist eine Position der jeweiligen Sensoreinheit S1, S2 derart gewählt, dass beim Tragen der brillenförmigen Trägereinheit TE die erste Sensoreinheit S1 z.B. annähernd beim linken Ohr und die zweite Sensoreinheit S2 annähernd beim rechten Ohr des Anwenders platziert ist.

Weiterhin kann beispielsweise in der brillenförmigen Trägereinheit TE eine Führungsrille vorgesehen sein, in welcher eine Verbindung VB von den zwei Sensoreinheiten S1, S2 und gegebenenfalls von dem Umgebungssensor US zur Auswerteeinheit AW angebracht werden kann. Die Auswerteeinheit AW kann ebenfalls an der Trägereinheit TE z.B. über eine Steckverbindung angebracht sein oder - wie beispielhaft in Figur 2 dargestellt - als eigenständige Einheit ausgestaltet sein.

Die beispielhaft dargestellt Auswerteeinheit AW kann beispielweise zum Auswerten der vom Umgebungssensor US erfassten Umgebungs- und/oder Objektinformation ein Objekterkennungsmodul OM aufweisen, welches die erfasste Umgebungs- und/oder Objektinformation in Bezug darauf auswertet, welche Objekte beispielweise erfasst wurden und/oder ob z.B. zwischen zumindest einem der mit dem Umgebungssensor US erfassten Objekte und dem Anwender einen Annäherung besteht. Dazu kann vom Objekterkennungsmodul OM beispielsweise bei Verwendung eine Objekterkennungssoftware eingesetzt werden bzw. eine Auswertung der Pixel der erfassten Umgebungs- und/oder Objektinformation durchgeführt werden, wenn der Umgebungssensor US einen optischen Sensor, insbesondere eine Kamera, zum Erfassen dieser Information aufweist.

Für die Ermittlung der Bewegungsinformation des Anwenders auf Basis der laufend von den zwei Sensoreinheiten S1, S2 erfassten Bewegungsparameter, wie z.B. Lagewerten, Beschleunigungswerten und Drehbewegungswerten, kann die Auswerteeinheit ein Beschleunigungsmodul BM aufweisen. Vom Beschleunigungsmodul M werden beispielsweise auf Basis der von der jeweiligen Sensoreinheit S1, S2 erfassten Beschleunigungswerte Sensoreinheiten-spezifisch Bewegungsinformationen ermittelt. Dabei werden die von der jeweiligen Sensoreinheit S1, S2 erfassten Beschleunigungswerten in einen "schwerlosen Raum" umgerechnet bzw. mit Hilfe der von der jeweiligen Sensoreinheit S1, S2 erfassten Lagewerte die Gravitation aus dem jeweiligen erfassten Beschleunigungswert herausgerechnet. Dann wird für jede Sensoreinheit S1, S2 eine Bewegungsinformation z.B. in Form eines Bewegungs- oder Beschleunigungsvektors (z.B. bestehend aus Betrag bzw. Vektorlänge und Bewegungswinkel) ermittelt, wobei dabei ein Mittelwert der Beschleunigungswerte je Sensoreinheit S1, S2 über eine vorgebbare Anzahl an Werten (z.B. die letzten 25 erfassten Werte) verwendet wird. Die Sensoreinheiten-spezifische Bewegungsinformation wird dann zu einer Gesamtbewegungsinformation zusammengeführt, welche angibt, ob der Anwender bzw. die Anordnung AN sich bewegt und in welche Richtung diese Bewegung durchgeführt wird. Anhand der von den Sensoreinheiten S1, S2 jeweils getrennt erfassten Drehbewegungswerten wird dann beispielsweise festgestellt, ob die ermittelte Bewegungsinformation, insbesondere der ermittelte Bewegungswinkel, für eine weiterführende Verarbeitung in der Auswerteeinheit AW verwendet wird oder nicht. Dazu wird ein Wert, welcher aus den Drehbewegungswerten der zwei Sensoreinheiten S1, S2 gebildet wird, mit einem vorgegebenen Grenzwert verglichen. Für diesen Wert werden z.B. zuerst Sensoreinheiten-spezifisch aus den Drehbewegungswerten, welche von der jeweiligen Sensoreinheit S1, S2 erfasst wurden, Mittelwerte (z.B. über die letzten 25 erfassten Werte) gebildet und dann aus den beiden Mittelwerten der Wert z.B. als arithmetisches Mittel berechnet. Nur bei Überschreiten des Grenzwertes durch den aus den Drehbewegungswerten gebildeten Wert wird die Bewegungsinformation weiterverwendet bzw. nicht auf den Wert Null gesetzt.

Das Objekterkennungsmodul OM und das Beschleunigungsmodul BM verarbeiten die vom Umgebungssensor US bzw. von den beiden Sensoreinheiten S1, S2 erfassten Informationen, Daten und/oder Parameter z.B. parallel und in Echtzeit.

Für die Aggregation und Weiterverarbeitung der vom Objekterkennungsmodul OM ausgewertete Umgebungs- und/oder Objektinformation und der vom Beschleunigungsmodul BM ermittelten Bewegungsinformation kann die Auswerteeinheit AW ein Aggregationsmodul AM umfassen, welches die Datenaggregation und Verarbeitung beispielsweise ebenfalls in Echtzeit durchführt und daraus Hinweise für den Anwender ableitet.

Weiterhin weist die in Figur 2 dargestellte, bevorzugte Ausführungsform der Anordnung AN eine Ausgabeeinheit AE auf, welche z.B. als akustische Ausgabeeinheit AE ausgeführt sein kann und z.B. einen Lautsprecherteil umfasst. Diese kann entweder an der Trägereinheit TE lösbar oder festverbunden angebracht sein oder als eigene Einheit ausgestaltet sein, wobei die Ausgabeeinheit AE über eine drahtgebundene oder drahtlose Kommunikationsverbindung zur Auswerteeinheit AW verfügt. Bei einer an der Trägereinheit TE angebrachten Ausgabeeinheit AE kann beispielsweise die Ausgabeeinheit AE oder zumindest der zugehörige Lautsprecherteil zumindest bei einer der zwei Sensoreinheiten S1, S2 und damit in der Nähe eines der Ohren des Anwenders positioniert sein. Über die Kommunikationsverbindung können von der Auswerteeinheit AW z.B. bei einem festgestellten Gefahrenfall oder festgestellter Kollisionsgefahr Steuersignale und Daten für eine Ausgabe von Hinweisen für den Anwender übertragen werden. Die Hinweise können dann z.B. verbal in Form von vordefinierten Audiotexten oder in Form von Warnsignalen dem Anwender über einen Lautsprecherteil der Ausgabeeinheit AE ausgegeben werden.

Ein wie in Figur 2 dargestellte Ausführungsform der erfindungsgemäßen Anordnung AN, aber auch alternative Ausführungsformen, bei welchen die Trägereinheit TE z.B. als Stirnband, helmförmig oder in einer anderen, insbesondere am Kopf einfach anbringbaren Form ausgeführt ist, kann beispielsweise im Industriebereich eingesetzt werden, um z.B. im Bereich der Produktentwicklung und Fertigung, vor allem bei der Mensch-Maschine bzw. Roboter-Kollaboration, zu helfen, Hindernisse zu erkennen und den Anwender der erfindungsgemäßen Anordnung vor diesen zu warnen und dadurch ein Verletzungsrisiko und Unfallrisiko zu reduzieren. Weiterhin kann die erfindungsgemäße Anordnung beispielsweise auch von sehbehinderten oder blinden Menschen verwendet werden, um sich z.B. in einer Umgebung zu orientieren oder Hindernisse in alltäglichen Leben insbesondere in Echtzeit zu erkennen.

## Patentansprüche

1. Anordnung zum Erkennen von Hindernisobjekten und zum Warnen vor Kollisionen mit Objekten, wobei die Anordnung (AN) an einem Körperteil, insbesondere einem Kopf eines Anwenders, angebracht werden kann, und die Anordnung (AN) zumindest umfasst:
- einen Umgebungssensor (US) zum Erfassen von Objekten in einer Umgebung des Anwenders;
- zwei unabhängige Sensoreinheiten (S1, S2) zum Erfassen von Bewegungsparametern des Anwenders;
- eine Trägereinheit (TE), an welcher zumindest der Umgebungssensor (US) und die zwei Sensoreinheiten (S1, S2) zum Erfassen von Bewegungsparametern angebracht sind;
- eine Auswerteeinheit (AW), welche dazu eingerichtet ist:
o eine mittels des Umgebungssensors (US) erfasste Umgebungs- und/oder Objektinformation auszuwerten;
o auf Basis der mittels der zwei Sensoreinheiten (S1, S2) erfassten Bewegungsparameter eine Bewegungsinformation des Anwenders zu ermitteln;
o und die ausgewertete Umgebungs- und/oder Objektinformation mit der ermittelten Bewegungsinformation zu aggregieren und daraus Hinweise für den Anwender ableiten; und
- zumindest eine Ausgabeeinheit (AE), welche derart eingerichtet ist, dass dem Anwender Hinweise ausgegeben werden können.

2. Anordnung nach Anspruch 1, ***dadurch gekennzeichnet, dass*** weiterhin die Auswerteeinheit (AW) dazu eingerichtet ist, ein Auswerten der erfassten Umgebungs- und/oder Objektinformation, eine Ermittlung der Bewegungsinformation des Anwenders aus den erfassten Bewegungsparametern, eine Aggregation der ausgewerteten Umgebungs- und/oder Objektinformation mit der ermittelten Bewegungsinformation und ein Ableiten der Hinweise für den Anwender in Echtzeit durchzuführen.

3. Anordnung nach einem der Ansprüche 1 bis 2, ***dadurch gekennzeichnet, dass*** die zwei Sensoreinheiten (S1, S2) zum Erfassen von Bewegungsparametern jeweils dazu eingerichtet sind, als Bewegungsparameter zumindest jeweils Lagewerte, Beschleunigungswerte und Drehbewegungswerte für die Ermittlung der Bewegungsinformation des Anwenders zu erfassen.

4. Anordnung nach Anspruch 3, ***dadurch gekennzeichnet, dass*** die Auswerteeinheit (AW) zum Ermitteln der Bewegungsinformation des Anwenders auf Basis der mittels der zwei Sensoreinheiten (S1, S2) erfassten Bewegungsparameter derart eingerichtet ist, dass auf Basis der Beschleunigungswerte, welche von jeder der zwei Sensoreinheiten (S1, S2) unabhängig voneinander erfasst werden, die Bewegungsinformation des Anwenders ermittelt wird, wobei mit Hilfe der von den zwei Sensoreinheiten (S1, S2) unabhängig voneinander erfassten Lagewerte eine Wirkung der Erdbeschleunigung aus den erfassten Beschleunigungswerten herausgerechnet wird, und dass die ermittelte Bewegungsinformation nur dann zum Aggregieren mit der ausgewertete Umgebungs- und/oder Objektinformation genutzt wird, wenn von einem Wert, für dessen Bildung jeweils zumindest ein von jeder der zwei Sensoreinheiten (S1, S2) erfasster Drehbewegungswert verwendet wird, ein vordefinierter Grenzwert überschritten wird.

5. Anordnung nach einem der Ansprüche 1 bis 4, ***dadurch gekennzeichnet, dass*** die Auswerteeinheit (AW) dazu eingerichtet ist, aus den mittels der zwei Sensoreinheiten (S1, S2) erfassten Bewegungsparametern wiederholt in einem vorgegebenen zeitlichen Abstand eine Bewegungsinformation des Anwenders zu ermitteln.

6. Anordnung nach einem der vorangegangen Ansprüche, **dadurch *gekennzeichnet, dass*** die zwei Sensoreinheiten (S1, S2) zum Erfassen von Bewegungsparametern derart auf der Trägereinheit (TE) angebracht sind, dass sie symmetrisch zu einer Mittelachse (M) der Trägereinheit (TE) positioniert sind.

7. Anordnung nach einem der Ansprüche 1 bis 6, ***dadurch gekennzeichnet, dass*** die Auswerteeinheit (AW) auf der Trägereinheit (TE) lösbar angebracht ist.

8. Anordnung nach einem der Ansprüche 1 bis 6, ***dadurch gekennzeichnet, dass*** die Auswerteeinheit (AW) als eigenständige Einheit ausgestaltet ist.

9. Anordnung nach einem der vorangegangenen Ansprüche, ***dadurch gekennzeichnet, dass*** der Umgebungssensor (US) und die zwei Sensoreinheiten (S1, S2) zum Erfassen von Bewegungsparametern über eine drahtgebundene Verbindung (VB) mit der Auswerteeinheit (AW) lösbar verbunden sind.

10. Anordnung nach einem der vorangegangenen Ansprüche, ***dadurch gekennzeichnet, dass*** die Ausgabeeinheit (AE) als akustische Ausgabeeinheit ausgestaltet ist, welche dazu eingerichtet ist, die Hinweise für den Anwender in Form von vordefinierten Audiotexten und/oder Warnsignalen auszugeben.

11. Anordnung nach einem der vorangegangenen Ansprüche, ***dadurch gekennzeichnet, dass*** die Ausgabeeinheit (AE) auf der Trägereinheit (TE) lösbar oder mit der Trägereinheit (TE) festverbunden angebracht ist.

12. Anordnung nach einem der vorangegangenen Ansprüche, ***dadurch gekennzeichnet, dass*** die Ausgabeeinheit (AE) über eine Kommunikationsverbindung mit der Auswerteeinheit (AW) verbunden ist.

13. Anordnung nach einem der vorangegangenen Ansprüche, ***dadurch gekennzeichnet, dass*** der Umgebungssensor (US) einen optischen Sensor, insbesondere Kamera und/oder Wärmekamera, umfasst.

14. Anordnung nach einem der vorangegangenen Ansprüche, ***dadurch gekennzeichnet, dass*** die Trägereinheit (TE) als brillenförmig, helmförmig oder als Stirnband ausgestaltet sein kann.
